# EUROPÄISCHE PATENTSCHRIFT

(11) **EP 0 712 626 B1**
(45) Veröffentlichungstag und Bekanntmachung des Hinweises auf die Patenterteilung: **09.03.2005**
(21) Anmeldenummer: 95117108.1
(22) Anmeldetag: 31.10.1995
(51) Int. Cl.: A61K 31/385

(54) **Verwendung von R,S-(+/-)-alpha-Liponsäure, R-(+)-alpha-Liponsäure, S-(-)-alpha-Liponsäure in reduzierter oder oxidierter Form oder der Metabolite sowie deren Salze, Ester, Amide zur Herstellung von Rheologica**
Use of R,S-(+/-)-alpha-lipoic acid, R-(+)-alpha-lipoic acid, S-(-)-alpha-lipoic acid in reduced or oxidized form or their metabolites as well as their salts, esters, amides for the manufacture of rheological agents
Utilisation de l'acide R,S-(+/-)-alpha-lipoique, R-(+)-alpha-lipoique, S-(-)-alpha-lipoique en forme réducée ou oxidée ou des métabolites ainsi que leurs sels, esters, amides pour la manufacture des agents rhéologiques

(30) Priorität: 08.11.1994 DE 4439477
(43) Veröffentlichungstag der Anmeldung: 22.05.1996
(60) Teilanmeldung: 03018232.3
(73) Patentinhaber: VIATRIS GmbH & Co. KG, 60314 Frankfurt am Main (DE)
(72) Erfinder: Conrad, Frank, Dr., D-65933 Frankfurt (DE); Henrich, Hermann-August, Prof. Dr., D-97082 Würzburg (DE); Geise, Wolfgang, Dr., D-97241 Dipbach (DE); Ulrich, Heinz, Dr., D-63843 Niedernberg (DE)

(56) Entgegenhaltungen:
- EP-A- 0 326 034
- EP-A- 0 661 051
- DE-A- 4 138 040
- DE-A- 4 420 102
- CHEMICAL ABSTRACTS, vol. 67, no. 25, 18.Dezember 1967 Columbus, Ohio, US; abstract no. 115722n, XP002022024 & VITAMINY PREDUPREZHDENII LECH. PREZHDEVREMENNOGO STARENIYA, INST. GERONTOL. AKAD. MED. NAUK. SSSR, 1966, Seiten 196-203, I.N.YAKOVLEVA: "Effect of lipoic acid on lipid metabolism and on cell permeability in the middle-aged and senile."
- BIOLOGICAL CHEMISTRY HOPPE-SEYLER, Bd. 375, Nr. spec.suppl. 1, 1994, Seite s55 XP000057502 M.HOFMANN ET AL.: "Alterations of red cell membrane fluidity and -SH groups due to hyperglycemic conditins, are counteracted by alpha-lipoic acid and dialysis"
- ARCH.BIOCHEM.BIOPHYS., Bd. 324, Nr. 1, 1995, Seiten 85-92, XP000570198 M.HOFMANN ET AL.: "Decrease of Red Cell Membrane Fluidity and -SH Groups Due to Hyperglycemic Conditions Is Counteracted by alpha-Lipoic Acid"
- INDIAN J. CLIN. BIOCHEM., Bd. 7, Nr. 2, 1992, Seiten 115-120, XP000570195 S. JAYANTHI ET AL.: "ALTERATIONS IN RAT ERYTHROCYTE MEMBRANE OARAMETERS DURING CALCIUM OXALATE LITHIASIS AND EFFECT OF DL ALPHA-LIPOIC ACID"
- WIEN. KLIN. WOCHENSCHR., Bd. 84, Nr. 18, 5.Mai 1972, Seiten 291-294, XP000570299 J.BRUCK ET AL.: "Zur Frage einer konservativen "Basistherapie" zerebrovaskulärer Erkrankungen"
- J.CEREB.BLOOD FLOW METAB., Bd. 12, Nr. 1, 1992, Seiten 78-87, XP000570501 J.H.M.PREHN ET AL.: "Dihydrolipoate Reduces Neuronal Injury After Cerebral Ischemia"
- DIABETE METABOL., Bd. 20, Nr. 2bis, 1994, Seiten 219-228, XP000571260 P.J.GUILLAUSSEAU: "TRAITEMENT PREVENTIF DE LA MICROANGIOPATHIE DIABETIQUE: BLOQUER LES MECANISMES PATHOGENIQUES"
- P.RÖSEN: "The role of anti-oxidants in Diabetes mellitus" 1992 , F.A.GRIES ET AL. XP002022023 * Seite 139 - Seite 152 *
- WPI/DERWENT, ACCESSION NUMBER 93-343770, XP002022022 & SU 1 769 865 A (MOTHER CILD HEALTH PROTECT RES INST) 23.Oktober 1992

## Beschreibung

Bei Durchblutungsstörungen gewinnt die Therapie mit rheologisch wirksamen Substanzen an Bedeutung, besonders, wenn eine Rückbildung von rheologischen Hindernissen nicht mehr möglich ist. Rheologisch wirksame Substanzen verbessern die Fließfähigkeit des Blutes. Überwiegt neben anderen Wirkungen auf das Kreislaufsystem die rheologische Komponente werden diese durchblutungsfördernden Substanzen als "Rheologica" bezeichnet (Radke et al, 1983). Eine gesteigerte Fließfähigkeit erhöht den Blutfluss durch die Entstrombahn (Mikrozirkulation) und damit auch die Sauerstoffversorgung des Gewebes. Das ist besonders der Fall, wenn die Blutgefäße bei pathologischer Ausgangslage nicht mehr weitergestellt werden können, also die vasomotorische Reserve erschöpft ist.

α-Liponsäure wird chemisch als 1,2-Dithiolan-3-Pentansäure 5-(1,2-ditiolan-3-yl)-Valeransäure 5-3-(1,2-Dithioanyl)-Pentansäure bezeichnet. Die α-Liponsäure besitzt ein chirales C-Atom und tritt in zwei enantiomeren Formen auf und kommt physiologisch in Pflanzen, in Bakterien sowie im Säugeorganismus vor. Sie besitzt die Funktion eines Coenzyms in mitochondrialen Multienzymkomplexen wie z. B. den der Pyruvatdehydrogenase, der α-Ketoglutaratdehydrogenase und der Dehydrogenase der verzweigtkettigen Aminosäuren. Im Stoffwechsel kann die α-Liponsäure von der oxidierten Form (Disulfidbrücke) in die reduzierte Dihydroform mit zwei freien SH-Gruppen überführt werden. Beide Formen haben eine ausgeprägte antioxidative Wirkung (z. B. Kuklinski et al., 1991; Packer, 1993). Das Redoxpaar Dihydroliponsäure/α-Liponsäure besitzt zudem metallchelatierende Eigenschaften. In der Bundesrepublik Deutschland wird α-Liponsäure seit 1966 als Arzneimittel zur Behandlung von Lebererkrankungen, bei Pilzvergiftungen sowie bei peripheren Polyneuropathien angewendet. Aus Kenntnis dieser antioxidativen Wirkung, wird in der DE-OS 4138040 A1 allgemein eine Verbindung die freie Radikale fängt und die Thiolfunktionen besitzt, genannt. Der Anspruch gilt für Lösungen für die Perfusion, Konservierung und Reperfusion von Organen und betrifft die hier vorliegende Erfindung nicht, weil über die bekannte antioxidative Eigenschaft hinaus hier eine neue Wirkung in einer neuen Anwendung gefunden wurde.

Das SU-Patent 1769865 A1 betrifft die Verwendung von u. a. α-Liponsäure zur phänomenologischen Verbesserung der Blutzirkulation und zwar die Vergrößerung des Blutstromes durch die großen Gebärmuttergefäße und die Verringerung der volumetrischen Parameter im Bereich des Corions bei Plazentainsuffizienz.

Ein Kombinationspräparat mit Ginkgo biloba - Extrakt auch zur Prophylaxe und Behandlung von Durchblutungsstörungen ist in der EP-OS 0 326 034 (Költringer, 1989) beschrieben.

Darstellung der medizinischen Ausgangsprobleme:
1. Haemorheologische Störungen führen z. B. bei Diabetikern zu einer Verschlechterung der Mikrozirkulation und zu Komplikationen im Rahmen des Diabetes. Die Prävalenz von Mikroaneurismen an den Nagelfalzkapillaren war bei Typ I Diabetikern mit und ohne retinalen Veränderungen signifikant gesteigert (Zaugg-Vesti et al., 1994).
2. Die periphere Verschlusskrankheit

Sie ist gekennzeichnet durch eine Störung des Blutflusses und der Gewebeperfusion.

Es bestand daher die Aufgabe für die als Folge gestörter rheologischer Bluteigenschaften, nämlich Viskosität des Blutplasmas, Viskosität korpuskulärer Bestandteile, Flexibilität, Adhesivität, Aggregabilität fester Bestandteile vor allem der Erythrozyten und Thrombozyten auftretenden Krankheitsbilder bei der Mikroangiopathie eine wirkungsvolle Therapiemöglichkeit zu schaffen.

Diese Aufgabe wird gelöst durch die Verwendung von R,S-α-Liponsäure, R-α-Liponsäure, S-α-Liponsäüre, R,S-α-Dihydroliponsäure, R-α-Dihydroliponsäure und S-α-Dihydroliponsäure, sowie deren Salze mit pharmakologisch akzeptablen Basen zur Herstellung eines Medikamentes zur Behandlung der Mikroangiopathie, insbesondere diabetischen Mikroangiopathie.

Gegenstand der Erfindung ist demnach vor allem die Verwendung zur Herstellung von Arzneimitteln enthaltend R,S-(+/s)-α-Liponsäure, R-(+)-α-Liponsäure, S-(-)-α-Liponsäure in reduzierter oder oxidierter Form sowie deren Salz mit pharmakologisch akzeptablen Basen zur Prävention und Therapie bei gestörter Mikrozirkulation bei Mikroangiopathie.

### Methode

Zum Nachweis der Wirksamkeit von α-Liponsäure und/oder ihren Enantiomeren, und/oder ihren Derivaten wurde die spezifische Methode der sinusoidalen oszillierenden Kapillar-Rheometrie verwendet (Chmiel H., 1990). Das viskoelastische Fließverhalten von Blut als modernstes Verfahren wird zur Bestimmung von pathologischen Veränderungen der Erythrozyten in der Klinischen Hämorheologie (z.B. bei arterieller Verschlußkrankheit, Schlaganfall und allgemein bei peripheren Gefäßkrankheiten) eingesetzt.

Zur Bestimmung der Viskoelastizität werden dynamisch rheologische Experimente durchgeführt, in denen zeitabhängig die Deformation und der Scher-Streß gemessen werden (sinusoidale oszillierende Scherversuche). Blut als nichtlineare viskoelastische Flüssigkeit zeigt eine Abnahme von η' und η'' bei ansteigender Amplituden der Scherung.
Für die hier unter "Beispiele" aufgeführten Messungen wurde das Oszillierende Kapillar-Rheometer, OCR-D (A. Paar, Graz, Austria) verwendet, bei dem die Methode auf einer gleichzeitigen Bestimmung der Volumenflußrate und des Druckgradienten entlang einer kreisrunden Glaskapillare beruht. Die Viskoelastizität kann so zwischen elastischen (Energiespeicherung) und viskösen (Energieverbrauch) Deformationen unterscheiden. Ansteigende Werte von η" beschreiben zunehmend elastischere Erythrozyten (weniger flexibel) mit Bildung von Aggregaten, die zu Störungen des Blutflußes in der Mikrozirkulation führen. Diese Eigenschaften sind verknüpft mit der Beschaffenheit der Zellmembran und den "bridging"-Mechanismen, die zu der o.g. Rouleaux-Bildung führen. Die Abnahme von η' bei höheren Scherraten kann von veränderten Orientierungen und Verlängerung der Erythrozyten sowie von einer Reduzierung der Energieverbrauchs herrühren. η' ist nicht nur vom Hämatokrit und der Plasmaviskosität abhängig, sondern auch vom Aggregationsverhalten und der elastischen Eigenschaft der Membran. Anhand der nachfolgenden Untersuchungen wird die Erfindung demonstriert:

### 1.) Ex vivo-in vitro-Untersuchungen

Mit menschlichem Blut und dessen Erythrozytenkonzentraten wurden Untersuchungen zur Viskoelastizität an der Universitätsklinik Würzburg bei Herrn Prof. Dr. Henrich durchgeführt.

Unter Lagerungsbedingungen wurde das Blut, versetzt mit alpha-Liponsäure, zeitabhängig aufbewahrt. Zu den verschiedenen Meßpunkten wurde das Blut im auto-logen Plasma resuspendiert und mit Phenazin-Methosulfat (PMS) versetzt, was die Ischämische Situation im Patienten nachempfindet (Methode; Maridonneau et al. 1983; Maridonneau-Parini und Harpey, 1985).

Die Dynamische Komponente der Blutviskosität, η'
Z. B. bei Einsatz von beiden Enantiomeren der alpha-Liponsäure kommt es zu einer ausgeprägten, meist hochsignifikanten Verringerung der Blutviskosität gegenüber der Kontrolle. Der relative Unterschied liegt bei 10 %.

Die Elastische Komponente der Blutviskosität, η''
Der relative Unterschied gegenüber der Kontrolle liegt hier bei 20 % Verbesserung.

### 2.) Tierexperimentelle Untersuchungen

Bei experimentellen Diabetes in Ratten wurde eine Verbesserung des perinervalen Blutflusses von Prof. Dr. Low, Mayo Clinic, Rochester, USA, gemessen. Die Durchblutung des peripheren Nervensystems der diabetischen Ratten war durch Thioctsäure um 50 % im Vergleich zur Kontrolle (nicht behandelte diabet. Tiere) gesteigert (p < 0,001).

### 3.) Untersuchung am Nagelfalz

7 Patienten mit diabetischer Polyneuropathie wurden 6 Wochen mit Thioctacid 2 x 600 behandelt. Es wurde am Anfang und am Ende eine kapillarmikroskopische Untersuchung am Nagelfalz durchgeführt. Im Rahmen der Untersuchung wurde ein Funktionstest durchgeführt, eine 3-minütige Ischämie mit anschließender Reperfusion. Zielparameter war die Zeit bis zum Erreichen der Re-perfusionsgeschwindigkeit. Vor der Behandlung betrug die Zeit 76,8 ± 25,2 sec, nach 6-wöchiger Behandlung 21,4 ± 8,1 sec.

Die Herstellung der Salze erfolgt in bekannter Weise (siehe auch Patentschrift EP-A 427 247). Die pharmazeutischen Zubereitungen enthalten im allgemeinen 5 mg bis 3 g der erfindungsgemäß verwendeten Verbindungen als Einzeldosis. Die erreichten Wirkspiegel im Körper sollen nach Mehrfachgabe vorzugsweise zwischen 0,1 und 100 mg/kg Körpergewicht liegen. Die Verabreichung erfolgt in Form von Tabletten, Kapseln, Granulaten, Kautabletten, Lutschtabletten, Pillen, Dragees, Brausetabletten, Brausepulver, Fertigtrinklösungen, flüssigen Formen zur parenteralen Applikation. Fertigtrinklösungen und flüssige Formen zur parenteralen Applikation können alkholische und wässrige Lösungen, Suspensionen und Emulsionen sein.

Bevorzugte Anwendungsformen sind zum Beispiel Tabletten, die vorzugsweise zwischen 5 mg und 2 g der erfindungsgemäß verwendeten Verbindungen sowie Lösungen, die vorzugsweise zwischen 1 mg und 200 mg der erfindungsgemäß verwendeten Verbindungen /ml Flüssigkeit enthalten.

Als Einzeldosen des Wirkstoffes sind beispielsweise zu nennen:
a. orale Formen: 10 mg - 3 g
b. parenterale Formen (intravenös oder intramuskulär): 10 mg - 12 g

Die Dosen a) und b) können beispielsweise 1- bis 6 mal täglich oder als Dauerinfusion gegeben werden.

### Beispiele zur Galenik

### Ausführungsbeispiele:

### Beispiel 1:

### Tabletten mit 100 mg R-(+)-α-Liponsäure

250 g R-(+)-α-Liponsäure werden mit 750 g mikrokristalliner Cellulose gleichmäßig verrieben. Nach dem Sieben der Mischung werden 250 g Stärke (Starch 1500/Colorcon), 732,5 g Lactose, 15 g Magnesiumstearat und 2,5 g hochdisperses Siliciumdioxid zugemischt und die Mischung zu Tabletten mit Gewicht 800,0 mg verpreßt.

Eine Tablette enthält 100 mg R-(+)-α-Liponsäure. Gegebenenfalls können die Tabletten nach üblichen Verfahren mit einem magensaftlöslichen oder magensaftpermeablen Filmüberzug versehen werden.

### Beispiel 2:

### Ampullen mit 250 mg R-(+)-α-Liponsäure als Trometamolsalz in 10 ml

250 g R-(+)-α-Liponsäure werden zusammen mit 352,3 g Trometamol (2-Amino-2-(hydroxymethyl)-1,3-propandiol) in einer Mischung aus 9 Liter Wasser für Injektionszwecke und 200 g 1,2-Propylenglykol unter Rühren gelöst. Die Lösung wird mit Wasser für Injektionszwecke auf 10 Liter aufgefüllt und anschließend durch ein Membranfilter der Porenweite 0,2 µm mit Glasfaservorfilter filtriert. Das Filtrat wird unter aseptischen Bedingungen zu 10 ml in sterilisierte 10 ml Ampullen abgefüllt.

Eine Ampulle enthält in 10 ml Injektionslösung 250 mg R-(+)-α-Liponsäure als Trometamolsalz.

### Beispiel 3:

### Ampullen mit 250 mg R-(-)-Dihydroliponsäure in 10 ml Injektionslösung

60 mg Trometamol und 1 g Ethylendiamintetraessigsäure, Dinatriumsalz werden in 1,8 Liter Wasser für Injektionszwecke gelöst. Die Lösung wird 30 Minuten lang mit Stickstoff begast. Unter weiterer Begasung mit Stickstoff werden in der Mischung 2 g Natriumdisulfit und anschließend 50 g R-(+)-Dihydroliponsäure gelöst. Die Lösung wird mit Wasser für Injektionszwecke, das mit Stickstoff begast wurde, auf ein Volumen von 2 Liter aufgefüllt. Nach sorgfältigem Mischen wird die Lösung über einen Membranfilter der Porenweite 0,2 µm filtriert und das Filtrat unter aseptischen Bedingungen und unter Vor- und Nachbegasung mit Stickstoff in Ampullen zu 10 ml Füllvolumen abgefüllt.

Eine Ampulle enthält in 10 ml Lösung 250 mg R-(-)-Dihydroliponsäure als Trometamolsalz

## Patentansprüche

1. Verwendung von R,S-α-Liponsäure, R-α-Liponsäure, S-α-Liponsäure, R,S-α-Dihydroliponsäure, R-α-Dihydroliponsäure und S-α-Dihydroliponsäure, sowie deren Salz mit pharmakologisch akzeptablen Basen zur Herstellung eines Medikamentes zur Behandlung der Mikroangiopathie.

2. Verwendung nach Anspruch 1 zur Herstellung eines Medikamentes zur Behandlung der diabetischen Mikroangiopathie.

## Claims

1. Use of R,S-α-lipoic acid, R-α-lipoic acid, S-α-lipoic acid, R,S-α-dihydrolipoic acid, R-α-dihydrolipoic acid and S-α-dihydrolipoic acid, and the salt thereof, with pharmacologically acceptable bases for producing a medicament for the treatment of microangiopathy.

2. Use according to Claim 1 for producing a medicament for the treatment of diabetic microangiopathy.

## Revendications

1. Utilisation d'acide R,S-α-lipoïque, d'acide R-α-lipoïque, d'acide S-α-lipoïque, d'acide R,S-α-dihydrolipoïque, d'acide R-α-dihydrolipoïque et d'acide S-α-dihydrolipoïque ainsi que de leurs sels avec des bases pharmacologiquement acceptables, pour la fabrication d'un médicament destiné au traitement de la microangiopathie.

2. Utilisation selon la revendication 1, pour la fabrication d'un médicament destiné au traitement de la microangiopathie diabétique.
